# EUROPEAN PATENT APPLICATION

(11) **EP 2 386 971 A2**
(43) Date of publication of application: **16.11.2011**
(21) Application number: 11177237.2
(22) Date of filing: 15.11.2007
(51) Int. Cl.: G06F 19/00

(54) **System and methods for diabetes management using consumer electronic devices**

(30) Priority: 17.11.2006 US 866409 P; 31.10.2007 US 931363
(62) Divisional of application: 07868762.1
(71) Applicant: Medtronic MiniMed, Inc., Northridge, CA 91325-1219 (US)
(72) Inventor: Patel, Hinanshu, Northridge, CA 91325 (US); Istoc, Emil, Winnetka, CA 91306 (US); Lin, Jack, Stevenson Ranch, CA 91381 (US); Narang, Ajit, North Hills, CA 91343 (US)
(74) Representative: Ruschke, Hans Edvard

(57) **Abstract**

A connector 110 for use with a consumer electronic device 100 and a medical device 120, the connector 110 comprises a connecting structure for attaching the connector 110 to the consumer electronic device 100, 200, 300, 400, 500; a power supply for providing power to the connector 110; a first communication protocol for transmitting data between the medical device 120 and the connector 110; and a second communication protocol for transmitting data between the connector 110 and the consumer electronic device 100. Also disclosed is a software-based application for receiving, managing and processing medical device data on a consumer electronic device, the software-based application comprising a graphical user interface for displaying data to a patient; an input mechanism for use by the patient to adjust settings in the software-based application; and alarms for alerting and reminding the patient. Furthermore there is disclosed a method of a managing diabetes using a consumer electronic device, including the steps of pairing a connector to a consumer electronic device; programming the consumer electronic device to communicate with a medical device for taking a physiological reading of a user, where the medical device is pre-programmed to communicate with the connector, allowing communication between the consumer electronic device and the medical device through the connector; sending data from the medical device to the consumer electronic device via the connector; and processing and displaying the data on the connector or on the consumer electronic device.

## Description

### RELATED APPLICATIONS

This application claims the benefit of prior filed U.S. Provisional Application Serial No. 60/866,409, filed on November 17,2006.

### FIELD OF THE INVENTION

Embodiments of the invention relate to diabetes management systems and, more particularly, to managing diabetes utilizing consumer electronic devices including cellular phones, MP3/digital audio players, personal digital assistants (PDAs), Smartphones, hybrid devices, and the like.

### BACKGROUND OF THE INVENTION

Infusion devices and glucose monitoring systems are relatively well known in the medial arts, particularly for use monitoring blood glucose levels and delivering or dispensing a prescribed medication to a user. In many cases, the user suffers from diabetes-a disease in which the body does not produce or properly use insulin. Approximately 13 million people in the United States have been diagnosed with some form of diabetes. Type 1 diabetes results from the body's failure to produce insulin. Type 2 diabetes results from insulin resistance in which the body fails to properly use insulin. In order to effectively manage and/or control the disease, diabetics must closely monitor and manage their blood glucose levels through exercise, diet and medications in addition to supplying their body with appropriate amounts of insulin based on daily routines. In particular, both Type 1 and Type 2 diabetics rely on insulin delivery and blood glucose monitoring systems to control diabetes.

External infusion devices have been used to deliver medication to a patient as generally described in U.S. Patent Nos. 4,562,751; 4,678,408; 4,685,903; 6,554,798, and 6,551,276 which are specifically incorporated by reference herein. In recent years, continuous glucose monitoring systems have been developed utilizing the latest sensor technologies incorporating both implantable and external sensors, as generally described in U.S. Pat. No. 5,391,250 entitled "Method of Fabricating Thin Film Sensors", U.S. Pat. No. 6,484,046 entitled "Electrochemical Analyte Sensor," and U.S. Pat. Nos, 5,390,671, 5,568,806 and 5,586,553, entitled "Transcutaneous Sensor Insertion Set," all of which are specifically incorporated by reference herein. Newer systems deliver the preciseness of finger stick measurements coupled with the convenience of not having to repeatedly prick the skin to obtain glucose measurements. These newer systems provide the equivalent of over 200 finger stick readings per day. Additionally, continuous glucose monitoring systems allow physicians and patients to monitor blood glucose trends of their body and suggest and deliver insulin based on each patient's particular needs. Accordingly, physicians and medical device companies are always searching for more convenient ways to keep diabetic patients aware of their blood glucose levels throughout the day.

Diabetic patients utilizing infusion therapy and continuous glucose monitoring systems depend on extremely precise and accurate systems to assure appropriate blood glucose readings and insulin delivery amounts. However, utilizing these forms of therapy requires the user to carry multiple medical devices containing intricate circuitry and processing capabilities. Although today's infusion devices and glucose monitoring systems are indeed compact, there remains a need in the art for more compact and/or converged systems to manage diabetes, such that the user's life style and mobility are not restricted.

### SUMMARY OF THE DISCLOSURE

According to an embodiment of the invention, a system is for managing diabetes using a consumer electronic device, including a medical device for taking a physiological reading of a user. The medical device includes a transmitter for communicating the physiological readings. In addition, the system includes a consumer electronic device, which includes software for managing and processing data obtained by the medical device. The system also includes a connector removably coupled to the consumer electronic device for facilitating communication between the medical device and the consumer electronic device. In some embodiments, the connector receives data from the medical device in a first communication protocol, and the connector transmits data to the consumer electronic device in a second communication protocol.

In other embodiments, the medical device is a continuous glucose monitoring system and/or an infusion device. In still additional embodiments, the consumer electronic device is a Smartphone. In still further embodiments, the consumer electronic device is an MP3 player. In some embodiments, the software on the consumer electronic device is a Java application.

In further embodiments, the Smartphone transmits the received data to a central server using an internet connection and/or transmits the received data to a different cellular phone using "Short Message Service" (commonly known as "SMS" or "text messaging"). In other embodiments, the Smartphone initiates a cellular phone call based on a particular event. In yet further embodiments, the software includes alarm capabilities to alert the user of a particular event. In other additional embodiments, the first communication protocol is a proprietary protocol maintained by the medical device manufacturer and the second communication protocol is Bluetooth.

According to another embodiment of the invention, a method is for managing diabetes using a consumer electronic device, including the steps of pairing a connector to a consumer electronic device. Next the consumer electronic device is programmed to communicate with a medical device for taking a physiological reading of a user, where the medical device is pre-programmed to communicate with the connector, allowing communication between the consumer electronic device and the medical device thorough the connector. Later, data is sent from the medical device to the consumer electronic device via the connector. The data is processed and displayed on the consumer electronic device. In some embodiments, the medical device is a continuous glucose monitoring system. In other embodiments, the consumer electronic device is a Smartphone.

According to a further embodiment of the invention, a system for providing information obtained from a medical device to an individual at a remote location is disclosed. The system includes a medical device for taking a physiological reading of a user, where the medical device includes a transmitter for communicating the physiological reading. The system also includes a local consumer electronic device, where the local consumer electronic device includes software for receiving, managing and processing data obtained by the medical device, A connector is also used by the system and the connector is removably coupled to the local consumer electronic device for facilitating communication between the medical device and the local consumer electronic device. Finally, a remote consumer electronic device is included for receiving information sent from the local consumer electronic device, where the connector receives data from the medical device in a first communication protocol, and the connector transmits data to the local consumer electronic device in a second communication protocol. In particular embodiments, the remote consumer electronic device receives information from the local consumer electronic device through a third communication protocol. In other embodiments, the first communication protocol is a proprietary protocol maintained by the medical device manufacturer, the second communication protocol is Bluetooth, and the third communication protocol is cellular communication. Still in additional embodiments, the cellular communication allows the local consumer electronic device to send information to the remote consumer electronic device using SMS, MMS, or email.

In yet another embodiment of the invention, a connector is for use with a consumer electronic device and a medical device. The connector includes a connecting structure for attaching the connector to the consumer electronic device and a power supply for providing power to the connector. The connector further includes a first communication protocol for transmitting data between the medical device and the connector and a second communication protocol for transmitting data between the connector and the consumer electronic device. In particular embodiments, the first communication protocol is a proprietary protocol maintained by a manufacturer of the medical device and the second communication protocol is Bluetooth.

According to another embodiment of the invention, a software-based application for receiving, managing and processing medical device data on a consumer electronic device is disclosed, In some embodiments, the software-based application includes a graphical user interface for displaying data to a patient, an input mechanism for use by the patient to adjust settings in the software-based application, and alarms for alerting and reminding the patient.

Other features and advantages of the invention will become apparent from the following detailed description, taken in conjunction with the accompanying drawings which illustrate, by way of example, various features of embodiments of the invention.

### BRIEF DESCRIPTION OF THE DRAWINGS

A detailed description of embodiments of the invention will be made with reference to the accompanying drawings, where like numerals designate corresponding parts or cross-sections in the several figures.

FIG. 1 is a simplified diagram of a diabetes management system including a PDA/Smartphone, a connector, and a glucose monitoring device in accordance with an embodiment of the present invention.

FIG. 2 shows perspective views of a PDA/Smartphone and connector in accordance with an embodiment of the present invention.

FIG. 3 is a simplified diagram of a diabetes management system including an MP3 player, a connector, and a glucose monitoring device in accordance with an embodiment of the present invention.

FIG. 4 shows perspective views of a PDA/Smartphone and connector in accordance with an embodiment of the present invention.

FIG. 5 shows the graphical user interface of the medical device software running on the consumer electronic device (BlackBerry) in accordance with an embodiment of the present invention.

FIG. 6 is a flow diagram describing operation of the diabetes management system using a BlackBerry, a connector and a blood glucose monitoring system in accordance with an embodiment of the present invention.

FIG. 7 shows screenshots of the software's graphical user interface (including menus and graphs) running on the consumer electronic device in accordance with an embodiment of the present invention.

FIG. 8 shows screenshots of glucose threshold profiles and menus on the consumer electronic device in accordance with an embodiment of the present invention.

FIG. 9 shows screenshots of the menu structure for the "Meter BG" sub-menu in accordance with an embodiment of the present invention.

FIG. 10 shows screenshots of the menu structure for the "Alarm History" sub-menu in accordance with an embodiment of the present invention.

FIG. 11 shows screenshots of the menu structure for the "Alerts" sub-menu in accordance with an embodiment of the present invention.

FIG. 12 shows screenshots of the menu structure for the "Sensor" sub-menu in accordance with an embodiment of the present invention.

FIG. 13 shows screenshots of the menu structure for the "Events" sub-menu in accordance with an embodiment of the present invention.

FIG. 14 shows screenshots of the software's indication profile menu in accordance with an embodiment of the present invention.

FIG. 15 shows a screenshot of a sample hypoglycemic alarm issued in the software running on the consumer electronic device in accordance with an embodiment of the present invention.

### DETAILED DESCRIPTION OF THE PREFERRED EMBODIMENTS

As shown in the drawings for purposes of illustration, the invention is embodied in a system for diabetes management including a medical device (MD) and a consumer electronic device (CED). The CED may be used to monitor and/or control the MD. In particular embodiments, the system may include a connector that plugs into the CED to allow communication between the MD and the CED. The medical device may be an external infusion device, implantable pump, glucose meter, glucose monitor, continuous glucose monitoring system, or the like. The CED may be any type of consumer electronic device including, but not limited to, cellular phones, personal digital assistants (PDAs), BlackBerry, Smartphones, pocketpc phones, mp3 players, radios, CD players, and the like. The CED may run its own proprietary operating system and would be capable of running a program to communicate and/or interact with the MD. The program may be Java based, web-based, contained on a digital memory card (SD, compact flash, microSD, or the like), and/or bundled on a CD-ROM with the MD. In particular embodiments, the program may work on multiple operating systems used on CEDs including, but not limited to, BlackBerry OS, Windows Mobile, Palm OS, .NET framework, Symbian, iPod OS, iPhone OS, Zune OS, and the like. Communication between the CED and the MD may be established using a connector that attaches to the CED. The connector would receive communication from the MD and relay that communication to the CED via a hard wired or wireless connection.

In particular embodiments, the connector may plug into an available port (i.e., mini-USB) on the CED (BlackBerry) and the connector may communicate with the BlackBerry via the mini-USB port or via Bluetooth communication. In these particular embodiments, a Java program would run on the BlackBerry to receive and control data received from the MD. In some embodiments, the MD may be a glucose sensor/transmitter of the type described in U.S. Patent No. 5,391,250 entitled "Method of Fabricating Thin Film Sensors", U.S. Patent No. 6,484,046 entitled "Electrochemical Analyte Sensor," U.S. Patent Nos. 5,390,671, 5,568,806 and 5,586,553, entitled "Transcutaneous Sensor Insertion Set," U.S. Patent No. 6,809,653 entitled "Telemetered Characteristic Monitor System And Method Of Using The Same," and U.S. Patent Application Serial Nos. 09/377,472, 11/225,790, 11/225,296, 11/322,568 and entitled "Telemetered Characteristic Monitor System And Method Of Using The Same," all of which are specifically incorporated by reference herein. The glucose sensor/transmitter would be capable of sending data to the connector using proprietary communication between the connector and the glucose sensor/transmitter. The data would then be sent to the BlackBerry and manipulated using the Java based program. The data obtained by the CED can show graphs, glucose trends, highs, lows, etc.-all accomplished using the familiar CED interface the user is accustomed to working with.

In further embodiments, the program may include alarm capabilities using the hardware and software available on, for example, a BlackBerry. Additionally, the data received from the glucose sensor/transmitter may be communicated to other locations using the BlackBerry's cellular capabilities. Data can be sent to other phones, central servers, and the like using "Short Message Service" (commonly known as "SMS" or "text messaging"). Data may also be sent to email addresses, other computers, and/or central servers using the BlackBerry's internet capabilities (GPRS, EDGE, EV-DO, 1xRTT, UMTS/HSDPA, Wi-Fi, WiMax, ZigBee, Wi-bro, and the like). In addition, based on certain conditions, the data may also provoke the BlackBerry to initiate a telephone call to emergency services (i.e., 911), a caregiver's house, a cell phone number, a central management station, or the like. In further embodiments, the Java program may include pre-recorded messages to playback upon initiation of a cellular telephone call to alert the person or computer receiving the call that a particular condition has been met. The Java program may also allow the user to perform event logging to associate particular events with glucose readings taken a certain time of day or during a particular activity (exercise, flying, driving, etc.). This data can then be uploaded to a web based diabetes management server like Carelink ® (managed by Medtronic MiniMed, Inc.). The data may also be sent to other servers, doctor's offices, parents, caregivers, or the like and insulin dosage recommendations may be made.

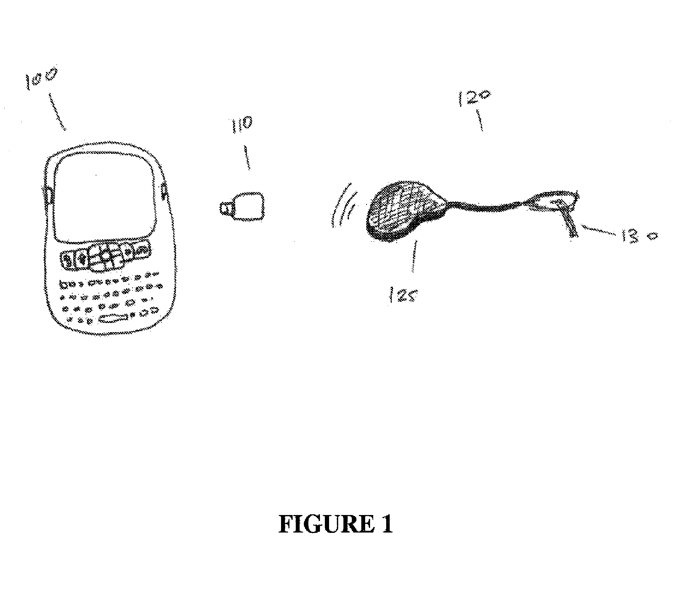

As shown in FIG. 1, a diabetes management system according to an embodiment of the invention includes a PDA/Smartphone 100, a connector 110 and a glucose monitoring device 120. The glucose monitoring device 120 includes a glucose transmitter 125 and a subcutaneous glucose sensor 130 of the type described U.S. Patent No. 5,391,250 entitled "Method of Fabricating Thin Film Sensors", U.S. Patent No. 6,484,046 entitled "Electrochemical Analyte Sensor," U.S. Patent Nos. 5,390,671, 5,568,806 and 5,586,553, entitled "Transcutaneous Sensor Insertion Set," U.S. Patent No. 6,809,653 entitled "Telemetered Characteristic Monitor System And Method Of Using The Same," and U.S. Patent Application Serial Nos. 09/377,472, 11/225,790, 11/225,296, 11/322,568 and entitled "Telemetered Characteristic Monitor System And Method Of Using The Same," all of which are specifically incorporated by reference herein. In particular embodiments, the connector 110 has a mini-USB connector that plugs into an available port on the PDA/Smartphone 100. Upon connection, the connector 110 allows communication between the PDA/Smartphone 100 and the glucose transmitter 125. The connector 110 may be used when a user chooses to use a glucose monitoring system 120 made by a different manufacturer from the PDA/Smartphone. For example, if a diabetic wanted to use the Guardian® RT Continuous Glucose Monitoring System developed by Medtronic MiniMed, Inc. with his BlackBerry PDA/Smartphone, the user would only need the appropriate connector 110 manufactured by Medtronic MiniMed that would plug into his BlackBerry. Upon connection, the Guardian® RT system would communicate with the user's BlackBerry.

The PDA/Smartphone 100 may be selected from any number of devices including the very popular BlackBerry® line of devices manufactured by Research in Motion. Other devices include the popular Treo devices produced Palm, Inc (Treo 600, 650, 680, 700w, 700p, 700wx, 750w), the HTC line of devices (AT&T 8525, AT&T Tilt, T-Mobile Dash, T-Mobile Wing, and the like), the Apple iPhone, and the like. Other devices and connectors are of the type described in U.S. Patent Nos, 6,558,320 and 6,641,533 entitled "Handheld Personal Data Assistant (PDA) with a Medical Device and Method of Using the Same," and U.S. Patent Application Serial No. 101429,385 entitled" Handheld Personal Data Assistant (PDA) with a Medical Device and Method of Using the Same," all of which are specifically incorporated by reference herein.

The connector 110 may be of the type described in U.S. Patent Application Serial No. 10/335,256 filed on December 12, 2002 and entitled "Relay Device for Transferring Information Between a Sensor System and Fluid Delivery System," which is specifically incorporated by reference herein. The connector 110 may include its own power supply or it may be powered through the connection to the CED. In some embodiments, the connector battery may charge its battery when plugged into the CED. The connector 110 allows communication between the CED and MD. In most situations, the MD will use a proprietary communication protocol that is associated with its monitor. However, the connector would communicate with the MD using the proprietary communication protocol and then relay the received data from the MD to the CED using any number of communication solutions. Such communication solutions include, but are not limited to, IR, RF, Bluetooth, wired connection via mini-USB, Wi-Fi, Zigbee, and the like.

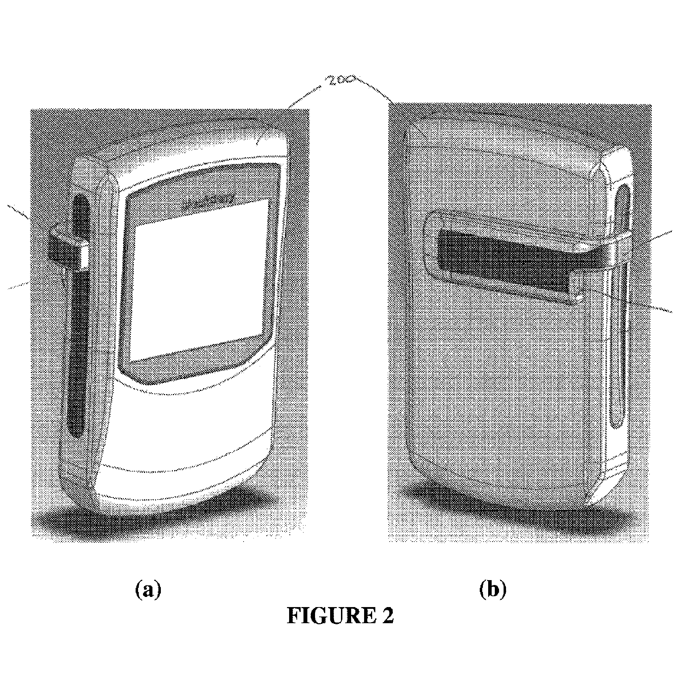

An example of a connector for a BlackBerry device is shown in FTGs. 2 and 4. FIG 2(a) shows a front perspective view of a BlackBerry device 200 with a connector 250 attached to the BlackBerry 200 mini-USB port. As shown in the figures, the connector 250 (or comlink) would have a small profile and fit nicely on the device to avoid changing the portable nature of the BlackBerry 200. Additionally, the connector 250 would include a male end that would plug into the BlackBerry 200 and a female end 255 that would allow the BlackBerry to continue its normal functionality-plug into a power outlet to charge, plug into a computer to synchronize data, etc. In particular embodiments, the connector 250 could communicate with the BlackBerry 200 using a wireless radio contained on the BlackBerry. In this aspect, a connector 250 can be attached to the BlackBerry 200 and can carry out protocol conversion. It can speak directly with the sensor transmitter's hardware to receive the data, and then it can send that data to the BlackBerry 200 using a standard communication protocol that the BlackBerry 200 can interpret (Bluetooth, IR, Zigbee, etc.) In other embodiments, the connector 250 communicates with the BlackBerry 200 via direct communication through the mini-USB port. For other devices that don't include a mini-USB port, the connector may be adapted to take the shape of an SD card to fit in a SDIO slot, or any other memory card shape to fit in the particular CED's available port (miniSD, microSD, memory stick, memory stick pro, memory stick duo, etc.).

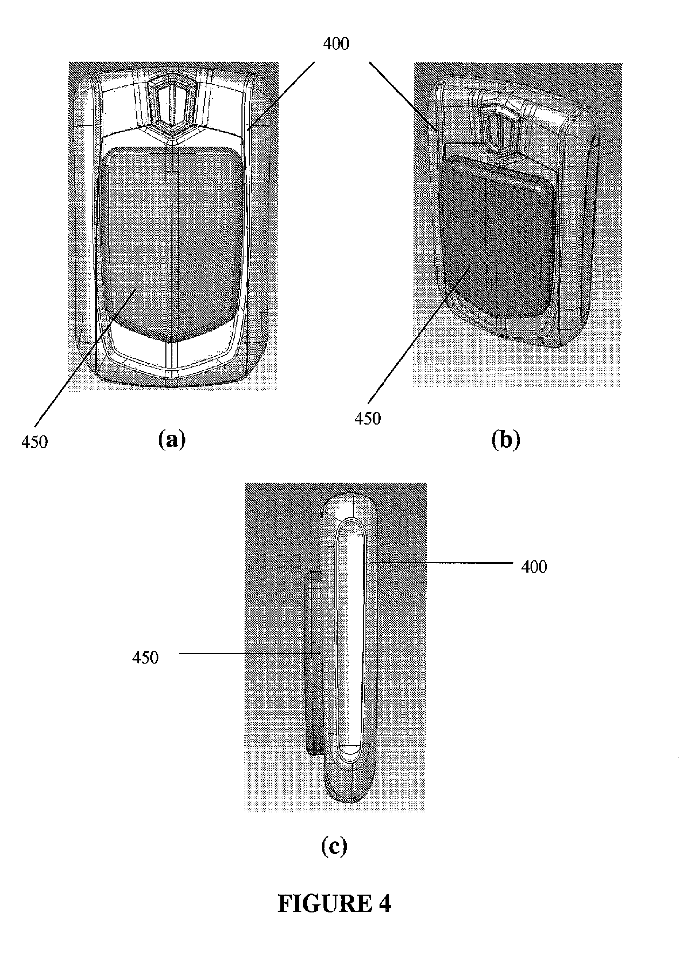

Another example of a connector is shown in FIG. 4. FIG. 4(a) shows a rear perspective view of the CED 400 with a connector 450 attached to the back of the CED. FIGS. 4(b) and (c) show other perspective views of the connector 450 attached to the back of the CED. In this example, the connector may be self charged with its own power supply, or may draw power from the battery pack installed in the BlackBerry. The connector 450 shown in FIG 4 need not utilize the mini-usb plug on the BlackBerry device. Instead, the connector 450 simply attaches to the back of the CED and remains self-powered.

The glucose monitoring device 120 may be replaced by any number of medical devices including, but not limited to a sensor with a built-in transmitter/receiver, a glucose monitor, a glucose meter, an insulin pump, and the like. In particular embodiments, glucose sensor 130 is a continuous glucose sensor capable of taking readings from a user on a continuous or near-continuous basis throughout the day. The glucose sensor transmitter 125 transmits blood glucose (BG) data to the connector 110 which is plugged into the CED 100. The CED 100 can take the BG data and display the information to the patient using the interface and control available on the CED 100 the user is already familiar with.

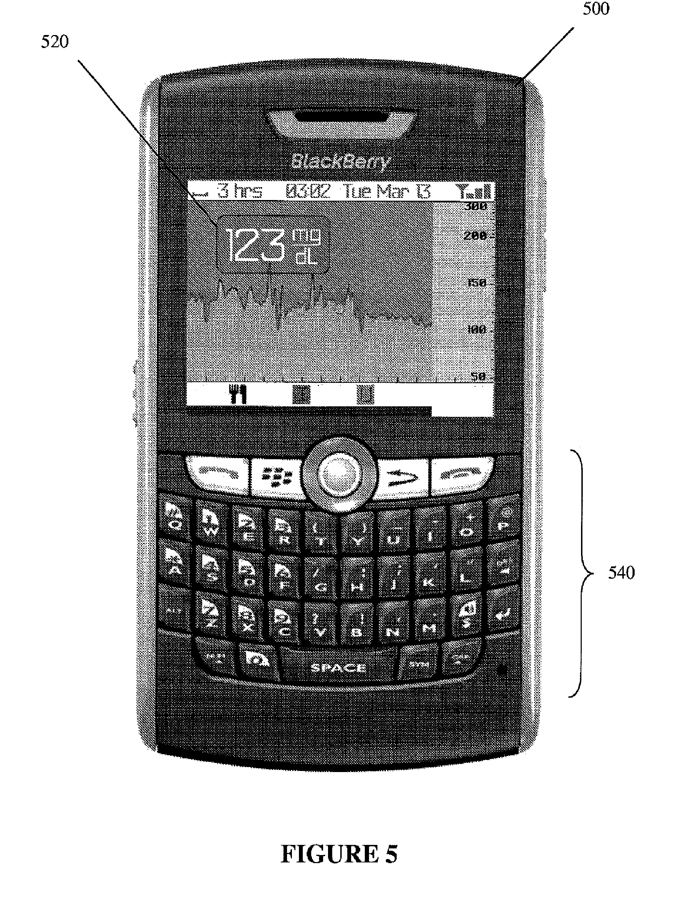

In these embodiments, software may be downloaded and/or preinstalled on the CED 100 to manipulate and manage the data received from the MD 120. The CED 100 may also include drivers to use the connector. In particular embodiments, the software will be written as a Java application-allowing the user to use their Java-enabled CED 100 to control and manage the data received from the MD 120. Java is a common mobile platform utilized by over 150 carriers. Currently, there are over 1.2 billion Java-enabled handsets and 8 out of 10 new phones shipped in 2005 were Java enabled. Additionally, there are 5 million Java developers worldwide. A Java application can be installed on any CED that includes Java capabilities (BlackBerry, T-Mobile Dash, Palm Treo, etc.). The installed Java application (as shown in FIG. 5) can display the BG data and can output the data in graphs, excel sheets, multi-day trackers, etc. Other programming options include, but are not limited to Windows CE, Windows Mobile, Palm Operating System, iPhone Operating System, .NET framework, Web-based Interface (no software needed to install, just run it from a supported browser, e.g. minimo, opera, pocket IE, Safari, etc.) Utilizing a web-based interface would allow seamless updates to the software without bothering the user on their CED.

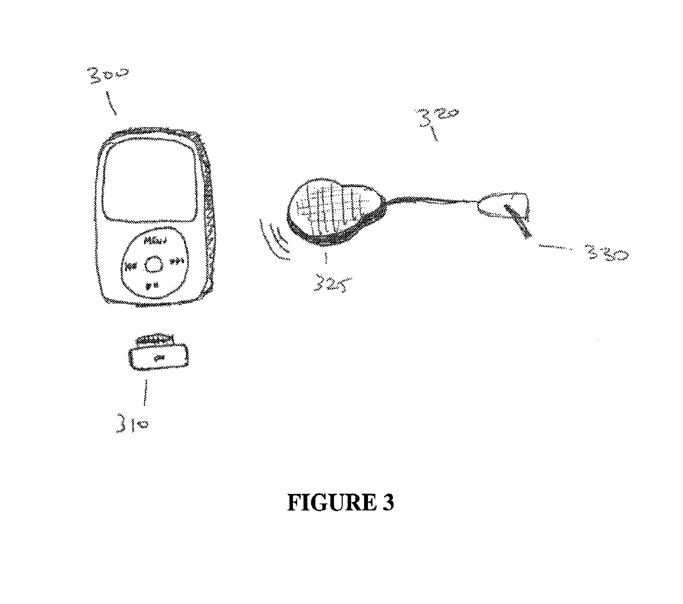

As shown in FIG. 3, a diabetes management system according to an embodiment of the invention includes an MP3 player 300, a connector 310 and a glucose monitoring device 320. The glucose monitoring device 320 includes a glucose transmitter 325 and a subcutaneous glucose sensor 330. In these embodiments, the iPod would be the CED of choice. The iPod 300 has become a world renowned device used by millions world wide. Allowing medical device management software to run on an iPod allows much more widespread use of important medical device technology-namely, the capability to receive and manage BG readings from a glucose monitoring system 320. The connector 310 may resemble the connector sold with the Nike+ iPod Sports Kit currently marketed by Nike and Apple. However, in some embodiments, the connector need not plug into the iPod connector and may communicate with the iPod and CED via wireless communication. The connector 310 would receive BG data from the glucose transmitter 325 and show the received data on the iPod 300. The iPod may be replaced by any number of MP3 and/or digital audio players including the Sansa, Zune, Gigabeat S, and the like.

In all embodiments described above, the BG data received by the CED can be manipulated by the software on the CED to show graphs, excel files, initiate reminders to check BG after a certain period of time and determine if BG readings are in a target range. In addition, software on the CED can track and manage BG information coupled with event markers inputted by the user.

In embodiments where the CED is a PDA/Smartphone, cell phone, or any other device with external communication capabilities, the CED may be configured to transmit the received BG data to external sources. For example, where the CED is a cellular PDA/Smartphone that includes cellular connectivity (voice and/or data) the software installed on the CED can transmit the received BG data (or any other data received from the MD) to the internet, caregivers, doctors, parents, and the like. The data can be transmitted via SMS, which has become a widely adopted communication mechanism all over the world. The software on the CED could be configured to send an SMS to the user's caregiver if a BG reading it too low, too high, or even if the user forgot to check his/her BG levels in for example, the last hour. In addition to SMS messages, the software on the CED may use the CED's internet connection to upload data to a central medical server or relay a message to a hospital, emergency room, or parent's email address. The communication to the internet may be accomplished using the CED's internet connection over Wi-Fi, GPRS, EDGE, 1xRTT, EV-DO, UMTS/HSDPA, or USB tethering. In addition, the software may use a CED's Bluetooth radio to send and/or synchronize data with the user's Bluetooth enabled PC and/or Bluetooth enabled automobile which has glucose sensing capabilities of the type described in U.S. Patent Application Serial No. 11/466,532 filed on August 23, 2006 and entitled "Automobile Glucose Sensor Monitoring System and Method of Using the Same," which is specifically incorporated by reference herein.

Utilizing the CED's wireless radios (cellular, Wi-Fi, Bluetooth, RF, Infrared, etc), the BG data may be sent to CEDs, other MDs, and/or uploaded to the internet to a Central Server. Once the data is on the central server, the server can manipulate the data and send information to other CEDs and other MDs, or send the information to a nurse or doctor. When the data is transmitted to any of the sources discussed above, secure communication may be achieved by encrypting the data.

In further embodiments, where the CED is a device with cellular capabilities (PDA/Smartphone, etc.), important information can be conveyed to others utilizing a cellular voice connection. In these embodiments, the software can provoke the CED to place a telephone call to selected phone numbers based on the event and/or situation at hand. The software may include pre-recorded messages that are played if a certain event takes place. Some events include out-of-range BG readings, imminent or current hypo- or hyperglycemic events, sensor calibration requirements, and the like. This particular feature may be useful for parents of diabetic children using continuous glucose monitoring systems.

In additional embodiments, the CED and MD would communicate directly with each other, without the need for a connector. In this case, the MD would include a standardized communication protocol compatible with the CED. Examples include: Wireless RF Communication (Bluetooth, Wi-Fi, ZigBee, etc.), Wireless IR Communication (irda, etc.), Wired communication using serial, usb, firewire, parallel, and the like. All functions would work similarly as described in the embodiments above.

In other embodiments, the software installed on the CED may also include an alarm function that utilizes the CED's hardware to alert and/or remind the user. Alarms may be included to notify the patient of potential crashes (hypo, hyper). Based on specific algorithms, the software can calculate predicted crashes based on BG trends. The user may be notified by an alarm included in the CED. Most PDA/Smartphones include some alert mechanism including auditory, tactile and visual that may be utilized by the software, The alarms may include differing sounds, colors, lights, vibrations, etc. In some embodiments, the alarm may be contained on the connector. If the connector has its own power supply, an alarm could be included to alert the patient of the above mentioned situations. In still other embodiments, the alarm may be included on the MD. The MD may send signals over to the connector and/or the CED to initiate an audio, tactile, or visual alarm.

The software installed on the CED may utilize multiple algorithms to manage and control the CED. A first algorithm may be a sensor data algorithm which allows the CED to obtain, store and display the BG data from the MD.

A second algorithm may be a patient event algorithm that associates a particular event during a particular time of day to a set of BG readings obtained from the sensor during that time period. The user can manually input their activity (*What event is taking place?*) and the software associates the subsequent BG readings with that particular event. Examples of events include eating (*what type of food?*), exercising, hot air ballooning, driving fast in a car, and the like. The events can be predefined by the software and/or customizable by the user. In addition, the software may ask the user of the event duration. If the event duration is known, then BG readings can be synchronized with the event. Using this event information, the BG values associated with that time period can be obtained and managed in a central server when the data is uploaded using any of the communication methods described above. With this information, doctors and medical analysts can determine effects on BG levels when patients participate in particular activities or eat particular foods. This data can be stored on the CED, the connector, or the MD and can subsequently be uploaded to a central server where all of the information can be managed (i.e., Carelink).

In still other embodiments, a food library can be created, maintained and updated using the event information. The food library can be stored on the CED, MD, and/or the connector. The user can constantly receive updates to the library via internet, SMS, etc. With an event logging algorithm, the food library may be maintained by scientists at a diabetes management facility. They can look at groups of patients and determine the effects certain foods and activities have based on user profiles. From there, "Results" are stored and accessible by other patients for reference purposes.

The software may also be capable of running Virtual Patient programs on the CED, MD and/or the connector. Some virtual patient programs allow patients and physicians to simulate BG responses based on a set of predefined variables.

In still further embodiments, the MD may be a continuous glucose monitoring system where the glucose sensor and glucose transmitter are fused into one device having a small profile. In addition, the glucose sensor/transmitter combination may perform all the processing of the BG data. Then the CED becomes a simple device that receives data and manipulates it. In still other embodiments, the CED may not be required because the glucose sensor/transmitter may have a display, controls, and wired/wireless connection to upload information to a central server, SMS functionality, and/or email capability.

FIG. 5 shows an example of the MD software 520 running on a CED 500 in an embodiment of the invention. In this particular figure, a BlackBerry (model 8800) is used to show the Java program running the MD software. As shows in FIG. 5, the software 520 displays information to the patient using the CED 500. This information includes current BG levels, the time of day, signal strength (of the cellular connection and/or of the connection to the MD), target blood glucose ranges and specific event markers. Each of these elements will be further described below. In addition, the patient can utilize the input functionality 540 of the CED 500 to setup and program their CED to control the MD. Input functionality 540 includes the BlackBerry's keyboard, scroll wheel, function keys, and the like.

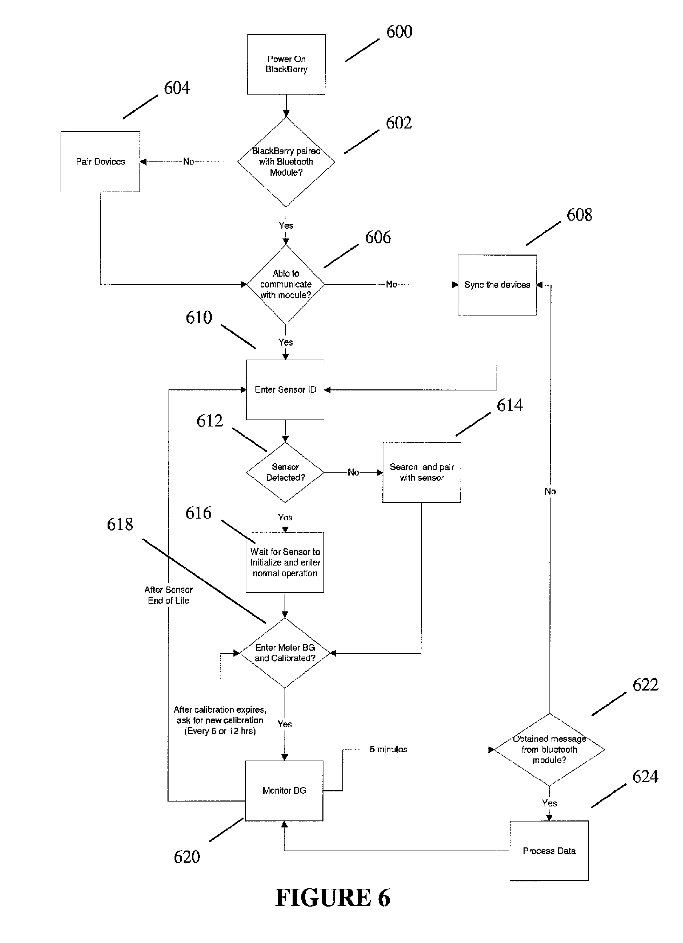

FIG. 6 shows a flow diagram describing the operation of a particular embodiment of the diabetes management system. In this example, a BlackBerry device serves as the CED and a glucose sensor functions as the MD. In step 600, the BlackBerry device is powered on. The BlackBerry must then be paired with the connector as described in step 602. If the connector is not yet paired, the user moves to step 604 and proceeds in pairing the connector with the BlackBerry. Standard methods of pairing Bluetooth devices are involved in the process. In particular, the connector may be placed in a Bluetooth discovery mode by holding down the power button on the connector. While the connector is in the discovery mode, the user must navigate to the Bluetooth connection menu in the BlackBerry and search for devices. Once the connector is found by the BlackBerry, the user will have to enter in a unique identifier code specific to their particular connector. The code should be found in the connector documentation. Once the correct code is entered, the connecter is paired with the BlackBerry.

Once the Bluetooth pairing between the BlackBerry and the connector is complete, the patient then moves on to step 606 to confirm communication with the connector. The sensor's ID must next be entered (step 610) into the BlackBerry and synchronized (step 608). After the unique sensor ID is entered (step 610), communication between the BlackBerry and the sensor (via the connector) is confirmed. If the sensor is detected in step 612, then the patient must wait for the sensor to initialize and enter normal operation (step 616). If the sensor in not detected, then the patient must search and pair with the sensor as described in step 614. After searching, pairing and initialization of the sensor in normal mode, the patient must then enter a blood glucose value obtained from a finger stick meter to calibrate the sensor. However, in some embodiments, this finger stick BG value may be excluded. In these embodiments, the sensor may have built calibration algorithms not requiring the finger stick BG value. In still further embodiments, the CED, MD and/or connector may include a built-in blood glucose meter for obtaining finger stick BG measurements.

After step 618 is complete, the glucose sensor monitor is now operational and the BlackBerry can receive the monitored BG values (step 620). As shown in the flow diagram, re-calibration may be needed after a certain amount of time has elapsed (i.e., every 3, 6, or 12 hours). If this is the case, the user will be reminded to re-calibrate and enter a new BG value. The patient may need to wait for a certain period of time for the sensor to re-calibrate (i.e,. 5, 10, 20 minutes). However, in some embodiments, there may be no waiting time necessary. In addition, after the glucose sensor has reached its end of life, a new glucose sensor must be obtained, and the sensor pairing process must take place again (step 610). In step 622, if a message is received from the connector (Bluetooth module), then the data may be processed (step 624). The data may be processed by the MD, the connector, and/or the BlackBerry.

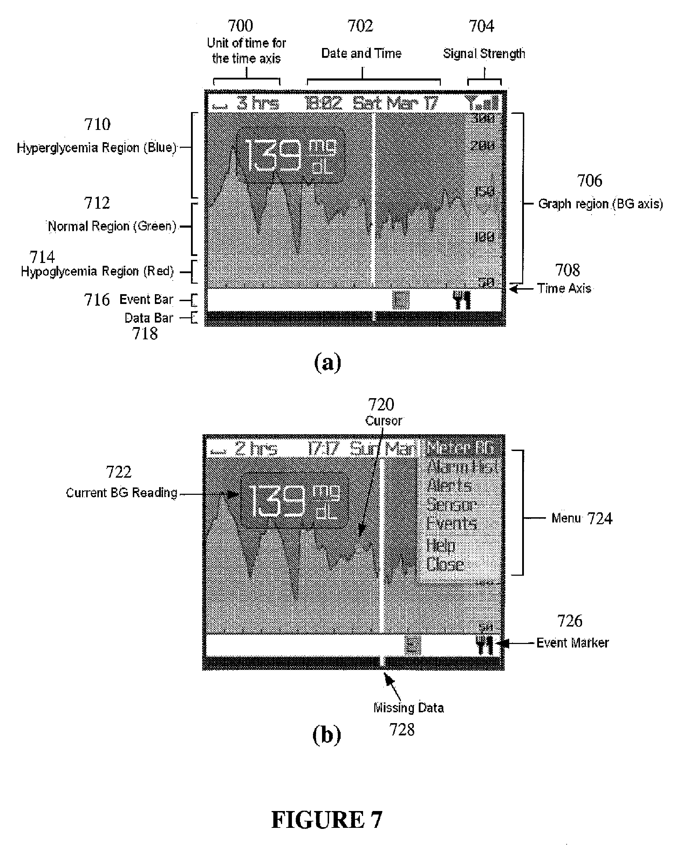

In FIG. 7, sample screenshots of the software's graphical user interface are shown. In these particular embodiments, FIG. 7(a) shows the GUI of the software running on the CED including a unit of time for the time axis (700); the date and time (702); and signal strength (704). The time axis 700 may be adjustable by the user and can range from displayed minute by minute increments to hourly, daily, weekly, monthly and/or yearly increments. The signal strength bars 704 may define the strength of the connection between the CED and the MD. In some instances, the user may toggle the indicator 704 to show the CED cellular signal connection also. The graph region 706 shows the blood glucose axis which defines ranges of BG levels. Range 710 shows a hyperglycemic region and is colored blue. The normal or target region 712 is defined by a green color indicating good control. The hypoglycemic region 714 is defined by a red color. The time axis is shown in 708 and, as described above, can be adjusted by the user for zooming in and out for a particular time ranges. Event bar 716 identifies specific markers placed by the patient and/or by the software to associate these values obtained during that time period with a specific event. Finally, data bar 718 confirms that BG data for a specific time period was received.

FIG. 7(b) points out the cursor 720 which is adjustable by the patient. The patient can move the cursor along the plot of data points using the CED's input function (keyboard, scroll wheel, arrow keys, and the like). Once the cursor 720 is highlighted, the patient's BG reading for the data point is shown in 722. The main menu 724 of the software is also shown in FIG. 7(b). The main menu 724 shows the patient his/her available options in customizing and accessing the various features of the software. A more detailed explanation of the various sub-menus is described below. Also, FIG. 7(b) shows an example of missing data 728 and how the data bar 718 shows a blank white space during the time no data was received. Event marker 726 shows in event bar 716 that a meal was eaten at that particular time. The graph shown in FIG. 7 describes one possible view of the GUI as used in an embodiment of the invention, However, in further embodiments, simpler or more complex GUIs may be used to provide the patient with more or less data. Simpler graphs may be used and/or no graphs may be shown. In some embodiments, the patient may customize the home screen of the software. Customizations may allow the patient to define specific variables, In even further embodiments, predefined screen layouts may exist on the software which allow doctors and clinicians to view more detailed graphs and charts (an "expert mode"). If an expert mode were included, an everyday "patient mode" might also be included utilizing some or all of the elements shows in FIG. 7.

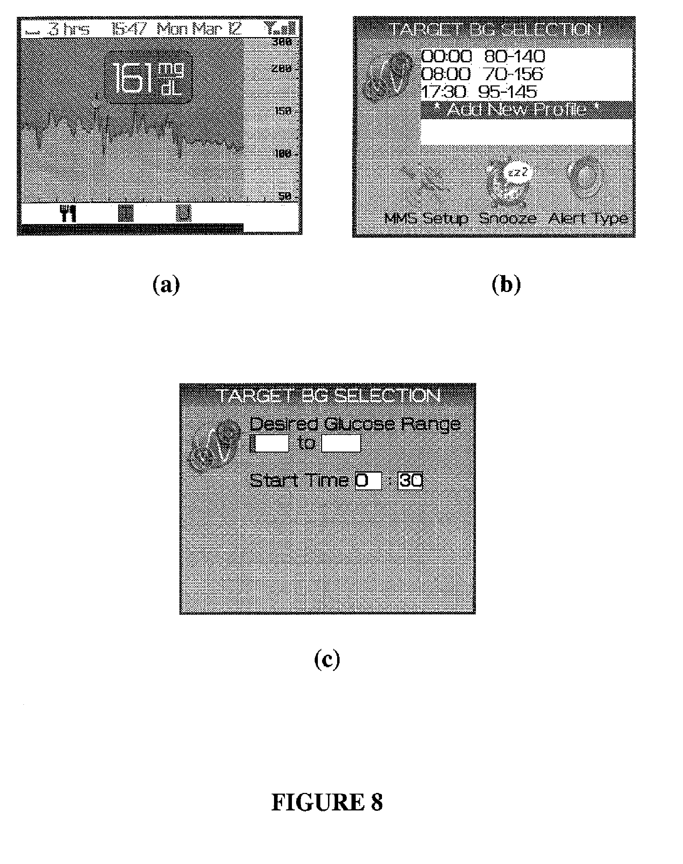

FIG. 8 shows screenshots of how the patient and/or doctor might set up target blood glucose threshold profiles for different times of day. As shown in FIG. 8(a), the ranges of blue (710), green (712), and red (714) regions fluctuate based on specific time periods. This function is useful since patients can sit down with their physician to determine what range their BG values should fall in during different times of the day (while sleeping, during work, in the morning, etc.). In addition, it also allows the patient to avoid extra alarms that would occur if there was only one specific range tied to each region. The more a patient understands his/her body, the better they will be able to define their BG threshold ranges. As shown in FIG. 8(b), the target BG selection screen allows the patient to add multiple profiles. In the screenshot shown in FIG. 8(b), the patient has entered three profiles: (1) From 0:00-8:00, threshold of 80 -140 mg/dL; (2) From 8:00 -17:30, threshold of 70 - 156 mg/dL; and (3) From 17:30 - 24:00 (0:00), threshold of 95 - 145 mg/dL. The patient may add additional profiles by clicking on the "Add New Profile" as shown in FIG. 8(b). The new profile screen is shown in FIG. 8(c), where the user enters the lower threshold, upper threshold, and start time of the profile. The end time is always the start of the next profile or 24:00 (0:00) if no sequential profile exists. When entering a start time, it must be 30 minutes after the previous profile. The user may also delete profiles which simply removes that profile. The graph on the main screen will be updated to display all profiles. In other embodiments, the patient may adjust the 30 minute value between profiles to better match his/her therapeutic needs. In some cases the timing may be longer or shorter based on each situation. In further embodiments, this value may or may not be adjustable by the patient.

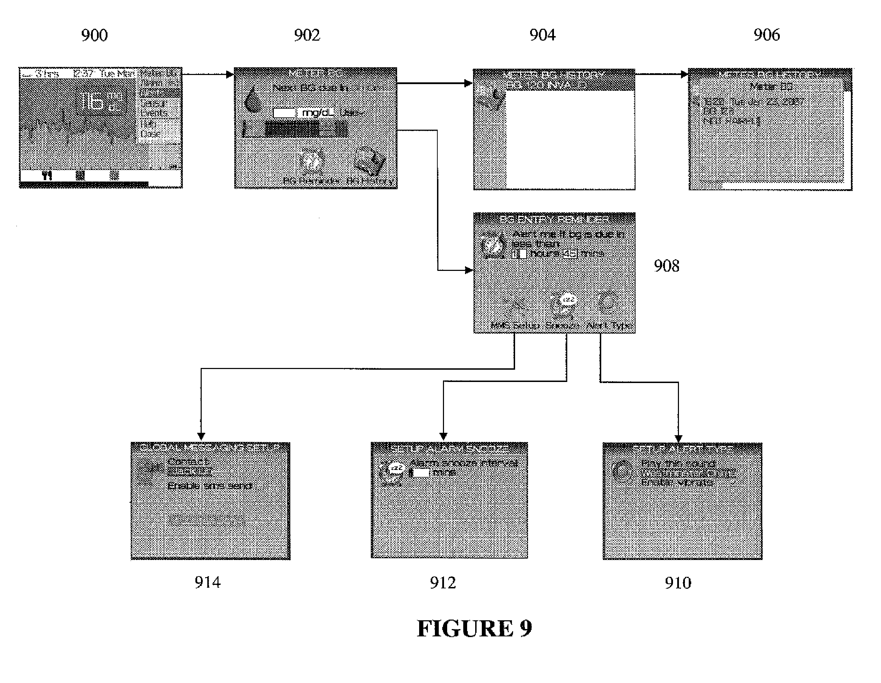

FIGs. 9-15 go through the various sub-menus of the functions available in the main menu 724 (FIG. 7(b)) of the software in particular embodiments of the invention. FIG. 9 shows the menu structure for the first heading under main menu 724-Meter BG (900). The "Meter BG" sub-menu is shown in 902. This menu allows the patient to enter in his/her current blood glucose obtained from a finger stick measurement. In addition, as shown in 902, a reminder is included under the first screen stating when the next BG reading is due. Two functions are included in sub-menu 902-BG Reminder and BG History. The BG History menu is show in 904 and it shows a log of the past BG values entered by the patient. Screenshot 906 shows the box that is pulled up when the patient highlights and selects a particular reading listed in screenshot 904. Again, in some embodiments, the meter BG readings are necessary to calibrate the sensor and assure proper and accurate sensor readings. In some cases, reading should be obtained every 3, 6, or 12 hours. However, in other embodiments, a meter BG value may only be required once a new sensor is utilized. In still further embodiments, no meter BG value is needed. Some embodiments may include a finger stick BG meter on the MD itself, built-in on the connector, and/or even built in to the CED.

Also shown in screenshot 902 is a BG Reminder button. The BG Reminder button pulls up the BG Reminder Entry sub-menu shown in 908. This allows the patient to configure a reminder/alarm to remind the patient that an upcoming BG value entry is required. The patient may choose any time frame. As shown in screenshot 908, the patient has entered a 1 hour and 45 minute reminder. The software may have predefined minimum and maximum values that are not adjustable by the patient to assure compliance. The range may be between 2 hours and 10 minutes. Other periods may range from 4 hours to 5 minutes, and the like. The BG Entry Reminder screen 908 also allows the patient to configure the indication mechanism with a choice between MMS/SMS Setup, Snooze, and Alert Type. The MMS/SMS setup screen 914 allows the patient to select a contact to receive an SMS reminding the patient or whichever contact is chose, that a BG value entry is due. The user may enter in a telephone number capable of receiving an SMS or select a contact already saved in the user's BlackBerry device. In addition, the software may also include a further menu allowing the patient to configure an automatic phone call to be placed to a specific contact in the event a BG value entry is due. More on this topic will be covered below in the hypo- and hyperglycemic alarms section.

The Setup Alarm Snooze screen 912 allows the patient to configure the snooze interval. Again, the software may include predefined and/or non-customizable time periods. But generally, the patient will be able to choose the timeframe. Finally, the Setup Alert Type screenshot 910 allows the patient to select an audio file to play when the reminder comes up. The files may be selected from audio files contained within the software itself (i.e., wav, midi, mp3, aac, aiff, m4a, and the like). In other embodiments, the patient may explore the BlackBerry device to select an audio file stored on the device's hard drive or external memory card. The patient may also have the software initiate a vibration alarm as well as an audio alert when the reminder is due. In further embodiments, a visual alert mechanism may also be utilized in the form of flashing LEDs or flashing screens. In some embodiments, the patient may pick and choose which type of alert mechanism he or she would like for each particular event reminder and/or alarm.

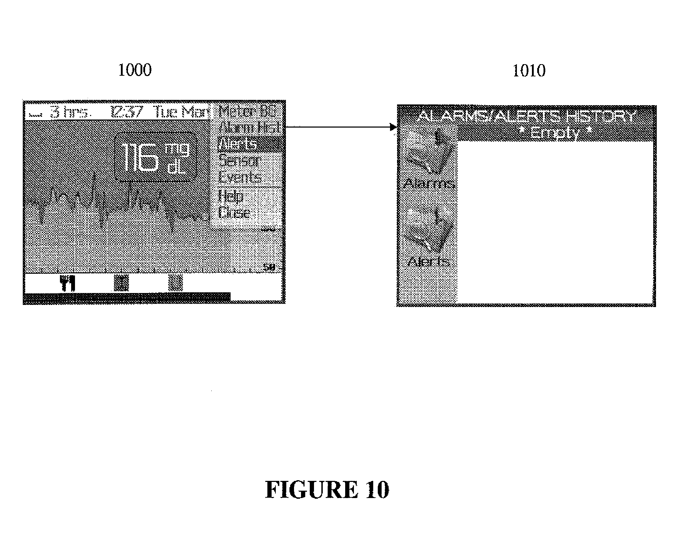

In FIG. 10, screenshot 1000 again shows the main menu (724) and the Alarm History sub-menu is shown in 1010. In this screenshot, the patient can view all previous alarms and alerts that were activated. Alarms and alerts can occur for simple reminders to take a meter BG reading, to more serious concerns of potential hypo- or hyperglycemic events or lost signal strength between the glucose sensor and the CED. The history screen is especially useful for doctors, caregivers and even parents who are monitoring their loved ones.

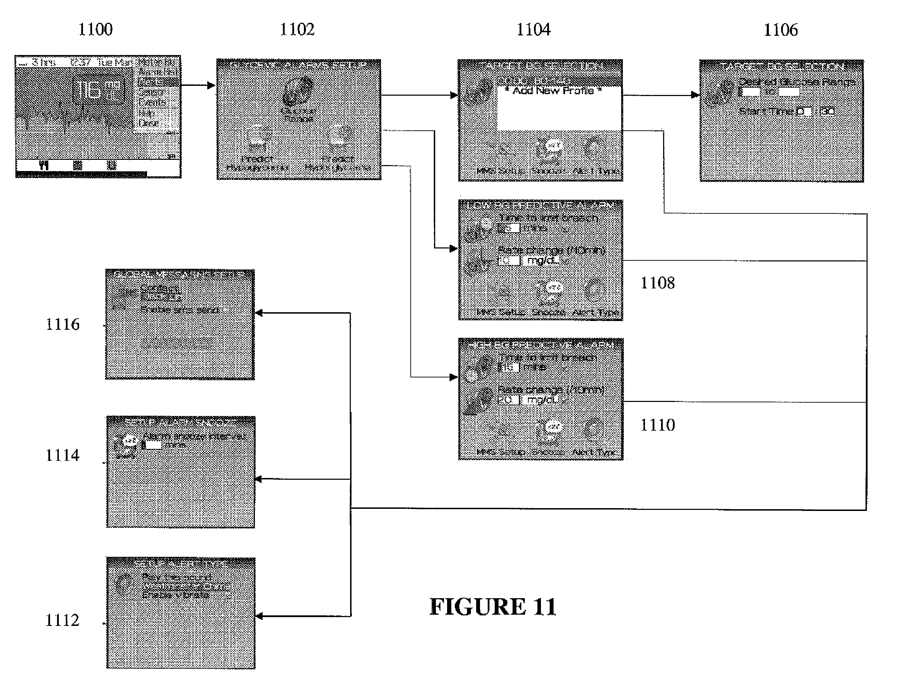

FIG. 11 shows the main menu in screenshot 1100 with the third highlighted sub-menu-Alerts. In sub-menu 1102, the patient can configure their Glycemic Alerts choosing from three separate categories-Glucose Range, Predict Hypoglycemia, and Predict Hyperglycemia. In the Target BG Selection screen 1104, the patient can set up his/her target BG values for various time periods throughout the day. Again, as described above, the patient enters the lower threshold, upper threshold, and start time of the profile as shown in screenshot 1106 (see also FIG. 8). In screenshot 1104, the patient can configure the indication mechanism as described above-via the MMS/SMS Setup screen (1116), Snooze screen (1114), and Alert Type screen (1112).

Screenshots 1108 and 1110 allow the patient to configure the Low BG and High BG predictive alarms. If the software determines that the patient's BG values are trending down or up and will fall outside the patient's target range, an alarm may issue. The patient can set up a Time To Limit Breach and a Rate of Change for both screens. Again, in some embodiments, the predictive alarms shown in 1108 and 1110 may be important alerts that doctors, caregivers and/or parents would like to be aware of. Accordingly, both alarms can be configured to send an SMS message (1116) or even initiate a telephone call to a phonebook contact or emergency service provide as discussed above. In some embodiments, the patient may access a different screen (not shown) to specify which contact should be called. In these screens, pre-recorded messages may be selectable so they can be played back to the recipient of the telephone call when a specific alert and/or alarm is activated. In additional embodiments, the patient may be able to configure the text contained within the SMS (or text message) sent to their phonebook contact or cellular number that is entered in screenshot 1116 or predefined text messages may be used. The algorithms of the predictive glycemic alarms may be of the type used in on-the-market insulin infusion devices and/or glucose monitoring systems.

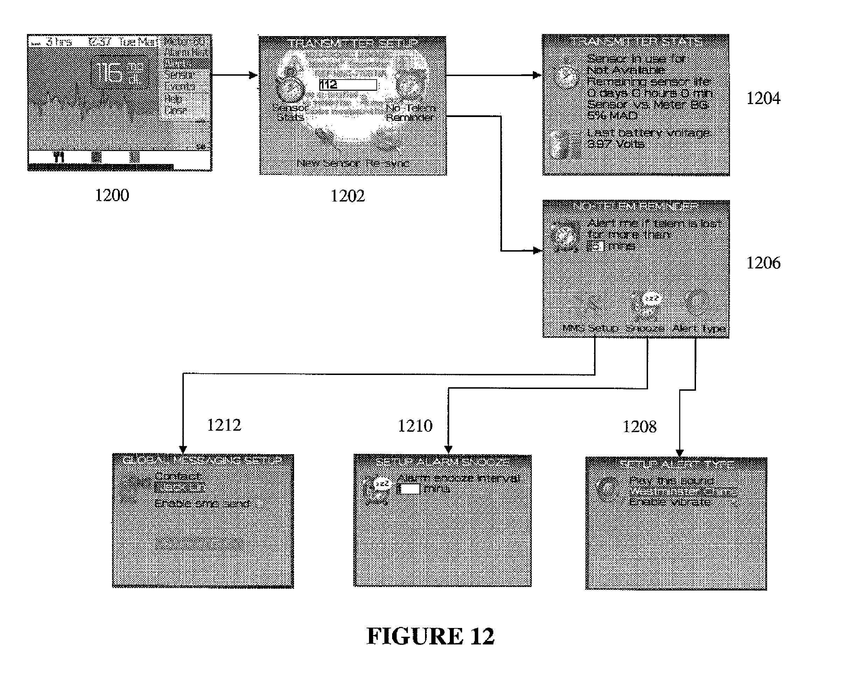

In FIG. 12, the patient accesses the main menu as shown in screenshot 1200 and selects sub-menu "Sensor". This takes the patient to the Transmitter Setup screen shown in 1202. The patient can review the sensor ID code, as well as re-synchronize the sensor or set up and pair a new sensor. In addition, the patient can review the sensor statistics as shown in screenshot 1204. Screenshot 1204 may provide the patient with sensor life information, sensor value discrepancy between recent BG meter readings, battery voltage, and the like. From the Transmitter Setup screen 1202, the patient may also access the No-Telem Reminder screen 1206. In screen 1206, the patient can configure a reminder alert if communication between the sensor and the connector is lost for more than X minutes. The alerts that issue may be configured as shown in screens 1208, 1210, and 1212.

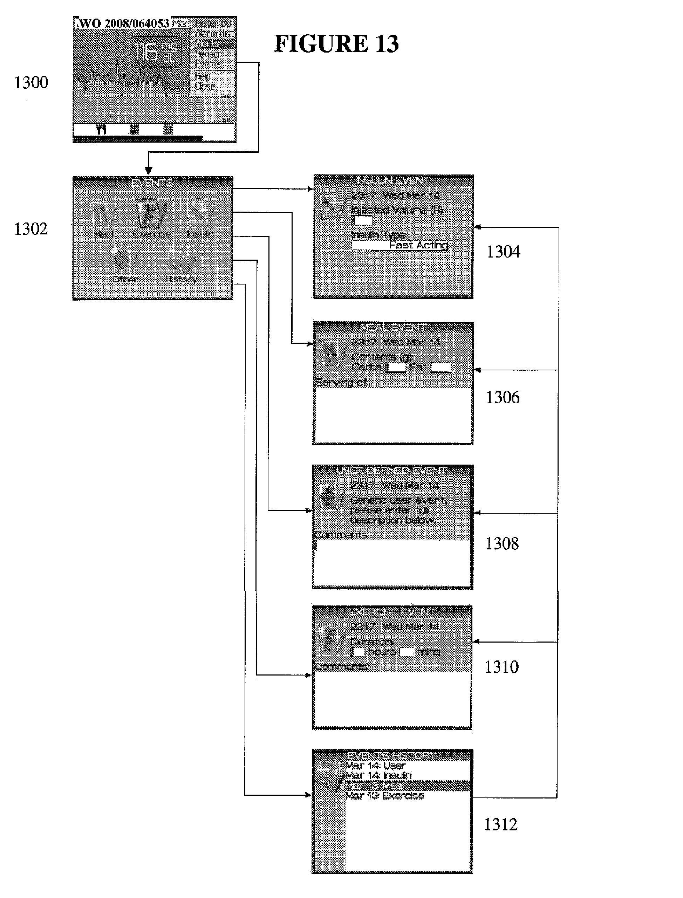

FIG. 13 shows the another sub-menu accessible by the patient in certain embodiments of the present invention. As shown in screen 1300, Events is the next selection and its screenshot is shown in 1302. Here the patient can configure the event markers as discussed above. In particular embodiments, the patient can choose from pre-defined events contained within the software and/or customizable events of varying duration. As shown in screen 1304, the patient can configure an Insulin Event to let the software know that a certain amount of insulin was administered at a particular time of day. Screenshot 1304 also allows input of the type of insulin administered (i.e., fast-acting, long-acting, inhalable, and the like). Screenshot 1306 allows the patient to configure a Meal Event market. Again, after the patient inputs the time and date, carbohydrate content and fat content can be entered. In yet additional embodiments, the software may include pre-defined food libraries from which the patient can select meals consumed. Further menus may allow the patient to select a predefined meal but customized to the patient's specific desire. Screenshot 1308 allows the patient to configure a User Defined Event. In this selection, the patient can enter in as much data as they feel appropriate to describe the specific activity taking place. When the data is uploaded to the diabetes management company and/or the patient's doctor, information can be obtained as to the effects on BG levels associated with the described activities. Screenshot 1310 allows the patient to configure an Exercise Event in terms of duration. In further embodiments, the patient may describe and/or choose from specific exercise activities including, but not limited to weightlifting, running, swimming, aerobics, yoga, and the like. Finally, screenshot 1312 pulls up an Events History screen where the patient can review previous events. In further embodiments, the patient may set up event markers before the event actually takes place. For example, if the patient works out every other day between 8:00am and 9:00am, the patient may set up an exercise event marker for those days beforehand. In further embodiments, the Events menu may include an indication mechanism selection to send data out to doctors, caregivers and/or parents regarding specific activities patients are participating in.

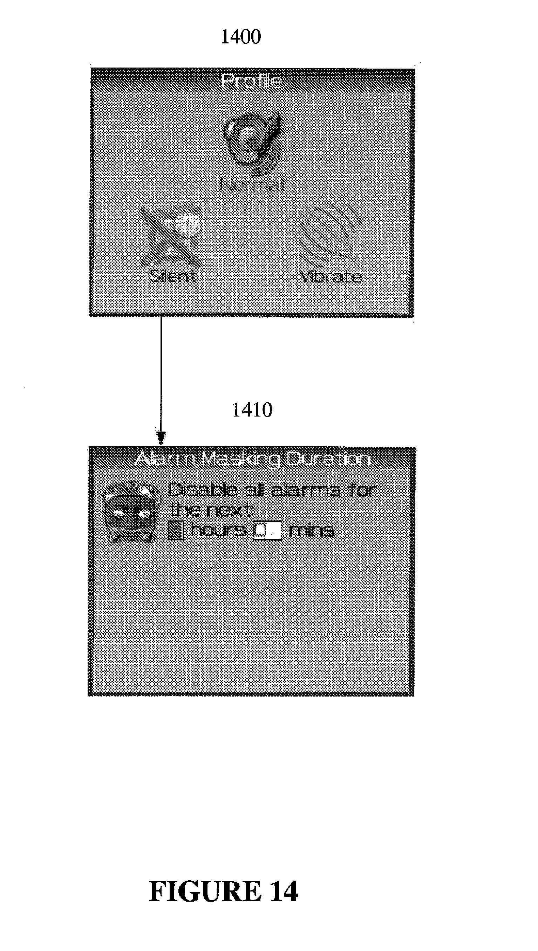

In FIG. 14, that patient can define the indication profile to be used on the CED. Screen 1400 shows the available options: Normal, Vibrate and Silent. The Normal profile will issue audio alerts based on specific alarms and reminders discussed above. The Vibrate profile issues tactile indications based on the same. In some embodiments, the user may select one or both profiles to occur simultaneously. The user may also select neither profile and, instead, may choose the Silent profile. Screenshot 1410 shows the sub-menu that is displayed when the Silent profile is selected. In particular, 1410 shows an Alarm Masking Duration menu where the patient enters a duration of time to disable upcoming alarms. This function may or may not be enabled in certain embodiments and may be customizable in other embodiments. In some cases, a parent who monitors his/her child may wish to disable this function entirely. Minimums and maximums may be predefined in the software and/or user selectable.

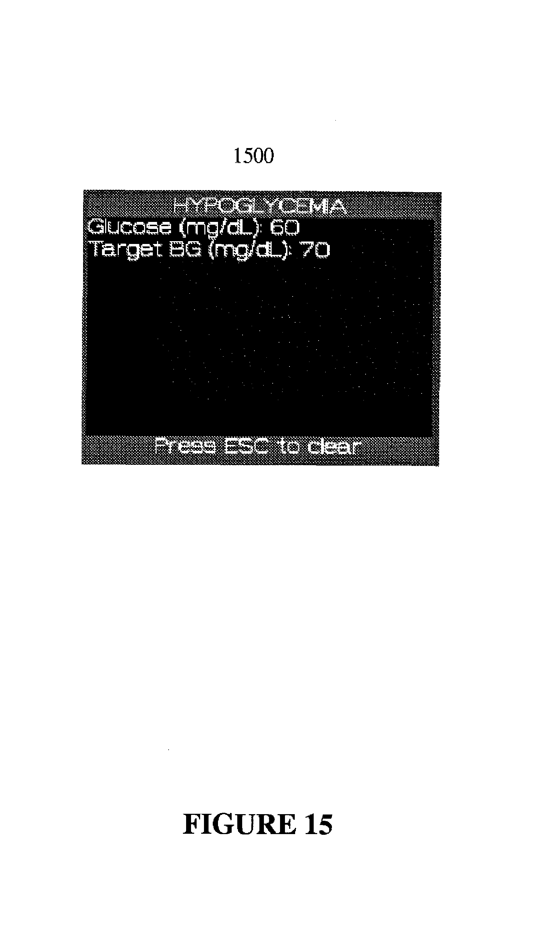

In FIG. 15, a sample screenshot 1500 is shown of a hypoglycemic alarm. In particular, the alarm may be accompanied by an audio and vibratory alert. The screen may display the name of the alarm (in this case, hypoglycemia). In addition, the activation of the alarm may indicate an SMS being sent to a loved one and/or telephone call being placed to emergency services as described above. In some embodiments, the patient may disable the alarm by acknowledging the indication. In other embodiments, certain alarms may not get dismissed until the patient does some corrective action as identified by the software.

The menu structure described in FIG. 9-15 describe a set of sample menus that may be included in embodiments of the diabetes management system. It shall be understood that additional and/or different menu screens may be included and/or excluded based on the particular CED, connector and MD components being utilized in the system. For example, if the CED is an MP3 player (i.e., the iPod Touch), different screen layouts and designs may be utilized in accordance with the above described embodiments utilizing the CEDs specific features (multi-touch touchscreen, accelerometers, proximity sensors, and the like). In further embodiments, the menu screens may be contained on the connector and not included on the CED at all.

While the description above refers to particular embodiments of the present invention, it will be understood that many modifications may be made without departing from the spirit thereof. The accompanying claims are intended to cover such modifications as would fall within the true scope and spirit of the present invention.

The presently disclosed embodiments are therefore to be considered in all respects as illustrative and not restrictive, the scope of the invention being indicated by the appended claims, rather than the foregoing description, and all changes which come within the meaning and range of equivalency of the claims are therefore intended to be embraced therein, Further embodiments envisaged are set out in the paragraphs below:
Paragraph 1. A system for managing diabetes using a consumer electronic device, the system comprising: a medical device for taking a physiological reading of a user, wherein the medical device includes a transmitter for communicating the physiological readings; a consumer electronic device, wherein the consumer electronic device includes software for managing and processing data obtained by the medical device; and a connector removably coupled to the consumer electronic device for facilitating communication between the medical device and the consumer electronic device; wherein the connector receives data from the medical device in a first communication protocol, and the connector transmits data to the consumer electronic device in a second communication protocol.
Paragraph 2. The system according to paragraph 1, wherein the medical device is a continuous glucose monitoring system.
Paragraph 3. The system according to paragraph 1, wherein the medical device is an infusion device.
Paragraph 4. The system according to paragraph 1, wherein the consumer electronic device is a Smartphone.
Paragraph 5. The system according to paragraph 1, wherein the consumer electronic device is an MP3 player.
Paragraph 6. The system according to paragraph 1, wherein the software is a Java application.
Paragraph 7. The system according to paragraph 4, wherein the Smartphone transmits the received data to a central server using an internet connection.
Paragraph 8. The system according to paragraph 4, wherein the Smartphone transmits the received data to a different cellular phone using SMS.
Paragraph 9. The system according to paragraph 4, wherein the Smartphone initiates a cellular phone call based on a particular event.
Paragraph 10. The system according to paragraph 1, wherein the software includes alarm capabilities to alert the user of a particular event.
Paragraph 11. The system according to paragraph 1, wherein the first communication protocol is a proprietary protocol maintained by the medical device manufacturer and the second communication protocol is Bluetooth.
Paragraph 12. A method for managing diabetes using a consumer electronic device, the method comprising the steps of: pairing a connector to a consumer electronic device; programming the consumer electronic device to communicate with a medical device for taking a physiological reading of a user, wherein the medical device is pre-programmed to communicate with the connector, allowing communication between the consumer electronic device and the medical device thorough the connector; sending data from the medical device to the consumer electronic device via the connector; and processing and displaying the data on the consumer electronic device.
Paragraph 13. The method according to paragraph 12, wherein the medical device is a continuous glucose monitoring system.
Paragraph 14. The method according to paragraph 12, wherein the consumer electronic device is a Smartphone.
Paragraph 15. A system for providing information obtained from a medical device to an individual at a remote location, the system comprising: a medical device for taking a physiological reading of a user, wherein the medical device includes a transmitter for communicating the physiological readings; a local consumer electronic device, wherein the local consumer electronic device includes software for receiving, managing and processing data obtained by the medical device; a connector removably coupled to the local consumer electronic device for facilitating communication between the medical device and the local consumer electronic device; and a remote consumer electronic device for receiving information sent from the local consumer electronic device, wherein the connector receives data from the medical device in a first communication protocol, and the connector transmits data to the local consumer electronic device in a second communication protocol; and wherein the remote consumer electronic device receives information from the local consumer electronic device through a third communication protocol.
Paragraph 16. The system according to paragraph 15, wherein the first communication protocol is a proprietary protocol maintained by the medical device manufacturer, the second communication protocol is Bluetooth, and the third communication protocol is cellular communication.
Paragraph 17. The system according to paragraph 16, wherein the cellular communication allows the local consumer electronic device to send information to the remote consumer electronic device using SMS, MMS, or email.
Paragraph 18. A software-based application for receiving, managing and processing medical device data on a consumer electronic device, the software - based application comprising: a graphical user interface for displaying data to a patient; an input mechanism for use by the patient to adjust settings in the software-based application; and alarms for alerting and reminding the patient.

## Claims

1. A connector (110, 110, 250, 310, 450) for use with a consumer electronic device (100,200, 300, 400, 500) and a medical device (120, 320), the connector (110, 110, 250, 310, 450) comprising:
a connecting structure for attaching the connector (110,110, 250, 310, 450) to the consumer electronic device (100, 200, 300, 400, 500);
a power supply for providing power to the connector (110, 110, 250, 310, 450);
a first communication protocol for transmitting data between the medical device (120,320) and the connector (110, 110, 250,310,450); and
a second communication protocol for transmitting data between the connector (110,110, 250, 310, 450) and the consumer electronic device (100, 200, 300, 400, 500).

2. A connector according to claim 1, wherein the first communication protocol is a proprietary protocol maintained by a manufacturer of the medial device and the second communication protocol is a different protocol, preferably Bluetooth.

3. A connector according to claim 1 or 2, further including an alarm to alert a patient of potential hypoglycemic and hyperglycemic crashes based on data received from the medical device (120, 320).

4. A connector according to any preceding claim, further including a store configured to hold data comprising blood glucose readings and events associated with sets of said blood glucose readings.

5. A connector according to any preceding claim, further including software capable of running a Virtual Patient program.

6. A connector according to any preceding claim, further including a built-in blood glucose meter for obtaining finger stick blood glucose measurements.

7. A connector according to any preceding claim, further including a display capable of showing menu screens.

8. A system comprising a connector according to any preceding claim connectable to a consumer electronic device to provide communication between an associated medical device and the consumer electronic device; and
a second connector also in accordance with any preceding claim, the second connector being connectable to the consumer electronic device to provide communication between a different associated medical device and the consumer electronic device.

9. A system according to claim 8, wherein the connection to the consumer electronic device is via a mini USB connector.

10. A software-based application for receiving, managing and processing medical device data on a consumer electronic device, the software-based application comprising:
a graphical user interface for displaying data to a patient;
an input mechanism for use by the patient to adjust settings in the software-based application; and
alarms for alerting and reminding the patient.

11. A method of a managing diabetes using a consumer electronic device, including the steps of pairing a connector to a consumer electronic device;
programming the consumer electronic device to communicate with a medical device for taking a physiological reading of a user, where the medical device is pre-programmed to communicate with the connector, allowing communication between the consumer electronic device and the medical device through the connector;
sending data from the medical device to the consumer electronic device via the connector; and
processing and displaying the data on the connector or on the consumer electronic device.

12. A method according to claim 11, whereby the medical device is a continuous glucose monitoring system.

13. A method according to claim 11 or 12, wherein the consumer electronic device is a Smartphone.

14. A method according to claim 11, wherein communication between the connector and the consumer electronic device is via Bluetooth.

15. A method according to claim 11, whereby the connection between the connector and the consumer electronic device is a removable coupling.
